## Europäisches Patentamt
## European Patent Office
## Office européen des brevets

(19)

(11) Publication number: **0 223 374**
**B1**

(12)

# EUROPEAN PATENT SPECIFICATION

(45) Date of publication of the patent specification:
**29.08.90**

(51) Int. Cl.⁵: **D04H 1/56, A61F 2/06**

(21) Application number: **86307631.1**

(22) Date of filing: **03.10.86**

(54) Improvements in electrostatically produced structures and methods of manufacturing thereof.

(30) Priority: **04.10.85  GB 8524541**

(43) Date of publication of application:
**27.05.87 Bulletin 87/22**

(45) Publication of the grant of the patent:
**29.08.90 Bulletin 90/35**

(84) Designated Contracting States:
**DE FR IT SE**

(56) References cited:
**DE-C- 658 721**
**GB-A- 1 530 990**
**GB-A- 2 120 946**
**GB-A- 2 121 286**
**GB-A- 2 142 870**

(73) Proprietor: **ETHICON INC., U.S. Route 22, Somerville New Jersey 08876(US)**
Proprietor: **THE UNIVERSITY OF LIVERPOOL, Mount Pleasant PO BOX 147, Liverpool L69 3BX(GB)**

(72) Inventor: **Berry, John Phillip, 5 Victoria Drive West Kirby, Wirral Merseyside L48 0QU(GB)**

(74) Representative: **Howick, Nicholas Keith et al, CARPMAELS & RANSFORD 43 Bloomsbury Square, London WC1A 2RA(GB)**

ACTORUM AG

## Description

The invention relates to electrostatically produced structures, for example tubular vascular grafts, and to methods of manufacturing such structures.

It has been proposed in the past to spin electrostatically fibrous structures of polymeric material such as polyurethane using an electrostatically charged, spinning mandrel as a fibre collector, a solution of the polymer being ejected towards the mandrel from capillary needles or other suitable means. The mandrel has in some circumstances been placed between charged electrodes to alter the electrostatic field created by the charged mandrel. The mandrel may be replaced by an alternative electrostatically charged collector if a tubular structure is not required.

It has been found that synthetic fibrous structures built up in this way generally have fibres of a diameter not larger than 1μm, and that the fibres are generally randomly orientated. Some directional bias can be induced by varying the speed of mandrel rotation, as indicated in our published British Patent Application Nos. GB-A-2121286 and GB-A-2120946. It is also known, from GB-A-2142870, that variation in anisotropy of an electrostatically spun graft can be achieved by varying the electrostatic field experienced by fibreizable material before collection by the mandrel. Such field variation is achieved using electrodes, as is disclosed also in DE-C-658721.

It has been found that the generally random nature of the fibrous structure and the small fibre size of 1μm or less has produced a tubular structure which can be prone to kinking and can therefore be a problem when used as an arterial graft particularly where limb movement is involved.

According to one aspect of the invention there is provided a tubular fibrous structure comprising small diameter fibres having a diameter in the range 0.5μm to 2μm and substantially larger diameter fibres having a diameter in the range 2μm to 15μm, said smaller diameter fibres being randomly oriented in the fibrous structure, said larger diameter fibres being embedded in a matrix of said small diameter fibres and said larger diameter fibres being generally oriented circumferentially with respect to the longitudinal axis of said tubular structure.

The structure may comprise a multiplicity of elongate voids extending generally circumferentially with respect to the longitudinal axis of the structure.

The structure may comprise an inner layer of fibres of the small diameter. The layer of fibres of larger diameter may be an intermediate layer, inside an outer layer of fibres of the small diameter.

The inner layer may be of a polymeric material adapted to be compatible with contact with blood, and may be of a thickness in the range 10μm to 60μm, preferably 40μm.

The intermediate layer may be of a thickness in the range 300μm to 2000μm.

The outer layer may be of a thickness in the range of 10μm to 60μm, preferably 40μm.

The tubular structure may have open areas or voids between the larger diameter fibres, said open areas or voids extending in the circumferential direction of the tubular structure whereby said structure has a low modulus in both compression and extension in the axial direction of said tubular structure.

According to a further aspect of the invention, there is provided a method of electrostatically spinning a tubular fibrous structure using an electrostatically charged, spinning mandrel and an electrostatically charged grid means in the region of the mandrel to produce an electrostatic field, which method comprises the steps of introducing into said electrostatic field a fibreizable material, collecting on said mandrel a first portion of said liquid in the form of fibres attracted directly to the mandrel and a second portion of said liquid in the form of fibres having been deflected towards said grid means to follow a longer path to said mandrel than the fibres from said first portion of liquid, whereby a tubular fibrous structure according to the invention is produced.

At least some of said second portion of said liquid may contact the grid means.

The method preferably comprises the step of selecting a desired potential for the mandrel and selecting a desired potential for the grid means prior to introducing fibreizable material into the electrostatic field produced by the mandrel and the grid means.

The grid means are preferably arranged such that the electrostatically charged surface of the grid means nearest the fibreizable material introduction means lies no nearer thereto than the mandrel. The electrostatic spinning process may be started with the mandrel at a first mandrel voltage and the grid means at a first grid voltage and the mandrel and the grid voltages varied to cause a variation in the diameter and orientation of at least a portion of the fibres forming the tubular fibrous structure.

The first mandrel voltage and the first grid voltage may be such as to produce fibres of a first diameter in the range 0.5μm to 2μm, generally randomly orientated, and the mandrel and grid voltages may be varied by increasing the electrostatic charge on the grid means relative to the mandrel to produce fibres of a larger diameter in the range 2μm to 15μm, as well as fibres of the first diameter, the fibres of larger diameter tending to be orientated generally circumferentially with respect to the longitudinal axis of the mandrel.

The method may include a further step of returning the mandrel to the first mandrel voltage and the grid means to the first grid voltage for a period at the end of the process.

The method may comprise the step of arranging and electrostatically charging the grid means such that a leading edge of the grid means lies forward of the mandrel with respect to the fibreizable material introduction means where fibres of said first diameter are required, and such that a leading edge of the grid means lies no nearer the fibreizable material introduction means than the mandrel when fibres of said second diameter are required.

The grid means may comprise a first pair of coplanar grids or plates on one side of the mandrel and a second pair of coplanar grids or plates on the other side of the mandrel and in a plane parallel to the first pair of coplanar grids or plates, one grid or plate of each coplanar pair lying forward of the mandrel with respect to the fibreizable material introduction means, and the other grid or plate of each coplanar pair lying no nearer the fibreizable material introduction means than the mandrel, and the method may comprise the steps of electrostatically charging both grids or plates of each pair when fibres of said first diameter are required and electrostatically charging the second grid or plate only of each pair when fibres of a diameter larger than said first diameter are required.

The mandrel may be charged to a voltage in the range 6 to 20kV, and an example of a preferred voltage for the mandrel, when of 4mm diameter, and the grid means is l2kV for the mandrel, 6kV for the grid means to produce fibres of the first diameter, 7kV for the mandrel, 9.2kV for the grid means when fibres of a larger diameter are to be produced. It will be appreciated, however, that these voltages are quoted merely by way of example as mandrel size, grid spacing and grid location have a fundamental effect on the voltage relationship between the mandrel and the grid means to produce different fibre production on the mandrel.

The tubular fibrous structure may have different fibrous structures at different locations along its length, and this feature may be achieved by traversing the fibreizable material introduction means along the length of the mandrel and varying the electrostatic potentials of the mandrel and the grid means as the fibreizable material introduction means moves relative to the mandrel to produce different electrostatic fields for fibre collection and have different fibrous structures at different axial locations on the mandrel. This variation may be conveniently controlled by a microprocessor programmed to repeat a desired sequence of electrostatic charge variation.

According to a further aspect of the invention, there is provided apparatus for electrostatically spinning a tubular fibrous structure, which apparatus comprises a mandrel to act as a collector for electrostatically spun fibres, means for electrostatically charging the mandrel and for varying the electrostatic charge thereon, means for rotating the mandrel, grid means in the region of the mandrel, means for electrostatically charging the grid means and for varying the electrostatic charge thereon, and means for introducing a fibreizable material into the electrostatic field, the surface of the grid means nearest to the fibreizable material introduction means lying no nearer thereto than the mandrel.

The grid means may comprise a pair of parallel plates or grids arranged on each side of the mandrel.

Leading edges of the plates or grids nearest the fibreizable material introduction means may be parallel, and the parallel leading edges may lie in a plane passing through or at least adjacent the mandrel.

The apparatus may further comprise secondary grid means extending forwardly of the grid means with respect to the fibreizable material introduction means. The secondary grid means may comprise a pair of parallel plates.

The apparatus may comprise means for electrostatically charging the grid means and the secondary grid means together, or the grid means alone.

The apparatus may further comprise microprocessor means for controlling variation of the voltages on the mandrel and the grid means in accordance with a desired sequence to produce a tubular fibrous structure having desired characteristics.

By way of example, one embodiment of a method of electrostatically spinning a tubular fibrous structure, of apparatus for carrying out the method and of a tubular fibrous structure will now be described with reference to the accompanying drawings, in which:-

Figure I is a side view of apparatus according to the invention for spinning a tubular fibrous structure in a first mode of operation;

Figure 2 is a side view illustrating the apparatus of Figure I in a second mode of operation;

Figure 3 is a scanning electron microscope photograph of the microstructure of the surface of a tubular structure including fibres of different diameters;

Figure 4 is a scanning electron microscope photograph of a section through the microstructure of a tubular fibrous structure having fibres of different diameters;

Figure 5 is a graph of the change in diameter of a tubular structure having fibres of different diameters as a function of internal pressure;

Figure 6 is a table of results of bending experiments on a fibrous structure having fibres of different diameter; and

Figure 7 is a photograph of a fibrous structure having fibres of different diameters undergoing a bend test.

Figures I and 2 illustrate apparatus for electrostatic spinning in two modes of operation.

Figures I and 2 illustrate the apparatus diagrammatically. The general electrostatic spinning process has been described in several Patent specifications already published, for example earlier published British application Nos. GB-A 2l2l286 and GB-A 2l20946.

The apparatus consists of an array of capillary needles l0 mounted on a carrier (not shown) with means for moving the carrier in reciprocating fashion parallel to a mandrel II. The means for moving the needle block is conveniently a motor.

The mandrel II in the embodiment illustrated is of 4mm diameter although it will be appreciated that other diameters may be used. There are means for rotating the mandrel at a variety of different speeds but typically the speed will be of several thousand revolutions per minute, a typical speed being 5000 r.p.m.

The capillary needles I0 are supplied with polymeric material such as polyurethane or other suitable polymeric material in solution (although it is possible to use a suspension) and material emanating from the needles is attracted towards the mandrel II by electrostatically charging the mandrel II to a potential of several kilovolts with respect to the needles I0. The process results in the production of fibres which collect on the mandrel II in a manner which has been described in specifications already published.

It has already been proposed to have electrostatically charged grids on either side of the mandrel II with respect to the needles I0 but the edges of the grids nearest the needles I0 have been significantly nearer the needles than has the mandrel. In the apparatus of Figures I and 2 pairs of plates I2, I3 and I4, I5 are provided. The plates I2, I3, I4 and I5 are conveniently of metallic sheeting although barred grids may be used if desired.

In the mode of operation illustrated in Figure I, all four plates I2, I3, I4 and I5 are electrostatically charged to a voltage less than the voltage to which the mandrel II is charged. Typical voltages for the spacing of the plates and the size of the mandrel are I2kV on the mandrel and 6kV on the plates I2, I3, I4 and I5. This results in an array of fibres leading from the needles I0 to the mandrel II substantially as shown in Figure I and is much the same as known electrostatic spinning processes.

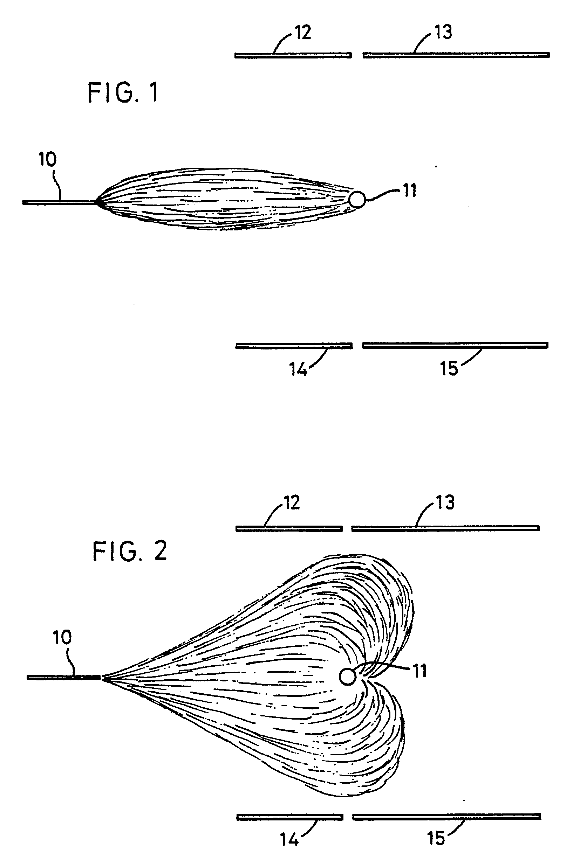

However, by changing the mode of operation to that shown in Figure 2, the fibrous structure formed on the mandrel II changes substantially. To produce the shape of fibre array emanating from the needles I0 shown in Figure 2, the forward plates I2 and I4 of the two pairs of plates are no longer electrostatically charged but are at the same potential as the needles I0. The rearward plates I3 and I5 having parallel leading edges which lie in a plane passing through or at least adjacent the mandrel II are charged to a higher voltage than was the case in Figure I and the mandrel voltage is reduced. This causes fibres to be attracted more readily towards the rearward plates I3 and I5 and thence to the mandrel II. Some fibres will be attracted directly to the mandrel II. Fine adjustment of the voltages is required as if the plates I3 and I5 are at too high a potential, material from the needles will simply be attracted to the plates I3 and I5 and not necessarily reach the mandrel II whereas if the mandrel voltage is too high relative to the plates I3 and I5, the fibrous structure will not differ significantly from that caused by the mode of operation described with reference to Figure I.

Typical voltages for the Figure 2 mode of operation are 9.2kV on the plates I3 and I5 and 7kV on the mandrel II. It will be appreciated, however, that the voltage relationship between the mandrel II and the plates I3 and will differ depending on the plate size and spacing, the mandrel diameter and the spacing of the needles I0 from the mandrel II and the plates I2, I3, I4 and I5.

The fibrous structure produced in the mode of operation described with reference to and shown in Figure 2 differs significantly from the fibrous structure produced by the mode of operation described with reference to and shown in Figure I. In the Figure I mode of operation, the fibres are generally in the range 0.5μm to 2μm, and mostly of approximately Iμm diameter, and are generally randomly orientated. In the Figure 2 mode of operation, however, while some fibres of diameter approximately Iμm (in the range 0.5μm to 2μm) are still formed on the mandrel II and their orientation random, fibres of a larger diameter are also produced, probably from the fibres whose path length to the mandrel II is lengthened because of the different electrostatic field. Fibres up to a diameter of I5μm may be produced and, in general, the larger the fibre diameter, the more likely is that fibre to be arranged circumferentially with respect to the longitudinal axis of the mandrel II. The larger diameter fibres are, in effect, more likely to be wound around the mandrel II leading to circumferential orientation.

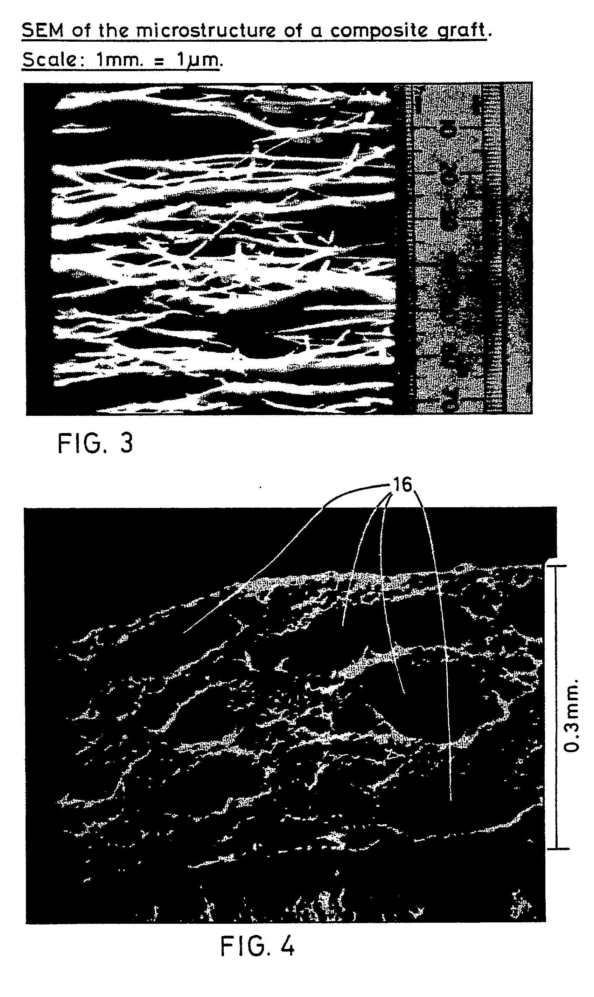

Figure 3 illustrates the type of microstructure produced in the Figure 2 mode of operation, it being apparent that fibres of a diameter of approximately Iμm extend randomly in direction over the structure whereas fibres of larger diameter extend generally circumferentially.

It has also been found that the microstructure produced in the mode of operation of Figure 2 includes elongate voids in the structure which generally extend circumferentially with respect to the longitudinal axis of the mandrel II and the tubular fibrous structure formed on it. Figure 4 illustrates the presence of the voids I6, Figure 4 being a scanning electron microscope photograph of a section through the microstructure. The presence of the voids I6 in the microstructure assists in resisting kinking of the structure upon bending.

It will be appreciated that tubular fibrous structures having varying fibrous structures across their cross-section can be produced readily by altering voltages at the mandrel II and plates I2, I3, I4 and I5 and by altering the mode of operation from the Figure I mode to the Figure 2 mode. It is also possible to feed solutions of different polymers to the needles I0 where different material characteristics are required. Thus a polymer having desirable characteristics for contact with blood and which may also be doped with, for example, a pharmaceutical can be introduced first of all in the Figure I mode to build up an inner layer for example of I0 to 60μm, preferably 40μm for contact with blood. Thereafter, simply by changing the polymer emanating from the needles I0 and the operational mode to that of the Figure 2 mode, the fibrous structure can be altered. Typically, the layer of the structure formed by the Figure 2 mode of operation would be between 300 and 2000μm thick. Finally, the Figure I mode could be used for an outer layer of perhaps I0μm to 60μm, preferably 40μm of the microstructure including fibres generally of Iμm diameter randomly orientated. Simply changing voltages can thus be used to create changes in the fibrous structure across the cross-section of the tubes and, once a desirable characteristic for a tube has been achieved, repeats can be readily made by microprocessor control.

Furthermore, it will be appreciated that tubular fibrous structures can be made with different characteristics along the length of the tubular structure by altering voltage or other characteristics as the needles I0 traverse along the mandrel II. This too can readily be controlled by a microprocessor.

A composite structure as described above including at least a layer of fibres including randomly orientated fibres of approximately Iμm diameter together with larger fibres tending to be orientated circumferentially around the tube has the appearance of a lightly ridged, flexible hose. The structure offers little resistance to bending and will assume a very tight loop without kinking. It has greater axial compliance than a similarly dimensioned tubular fibrous structure made entirely in the Figure I mode of operation but, most importantly, when compressed along its axis, it shortens with a minimal tendency to buckle. Mechanical tests have been carried out to quantify these features of the graft.

COMPARISON OF HOOP-AXIAL MODULI

A simplified tensile test was made on specimens cut from a tubular structure made in the Figure 2 mode of operation and from a tubular structure made from a Figure I mode of operation. The Young's modulus of the "dogbone" shaped specimens was measured and the results are given below:-
hoop modulus - Fig. I mode $5.4 \times 10^5$ Pa
hoop modulus - Fig. 2 mode $20.1 \times 10^5$ Pa
axial modulus - Fig. I mode $9.7 \times 10^5$ Pa
axial modulus - Fig. 2 mode $2.8 \times 10^5$ Pa

Compared with a Fig.I mode structure, the circumferential modulus of a Fig. 2 mode structure is increased by a factor of four and the axial modulus is decreased by a similar factor. This shows that the preferential fibre alignment in the Fig. 2 mode structure significantly changes the hoop/axial ration of moduli which explains in part the good bending properties. These results are consistent with the fibre orientation observed by scanning electron microscope for a Fig. 2 mode structure.

The static compliance of one of the Fig. 2 mode structures has been measured using a specimen 7cm in length, 3.7mm bore and 0.57mm wall thickness. The structure was pre-clotted with gelatin prior to being tested and was subjected to I5% axial strain. The internal pressure was ramped to 2000mm Hg and slowly brought back to zero, during which the change in diameter was repeatedly measured. The experiment was repeated at 20% and 25% axial strain.

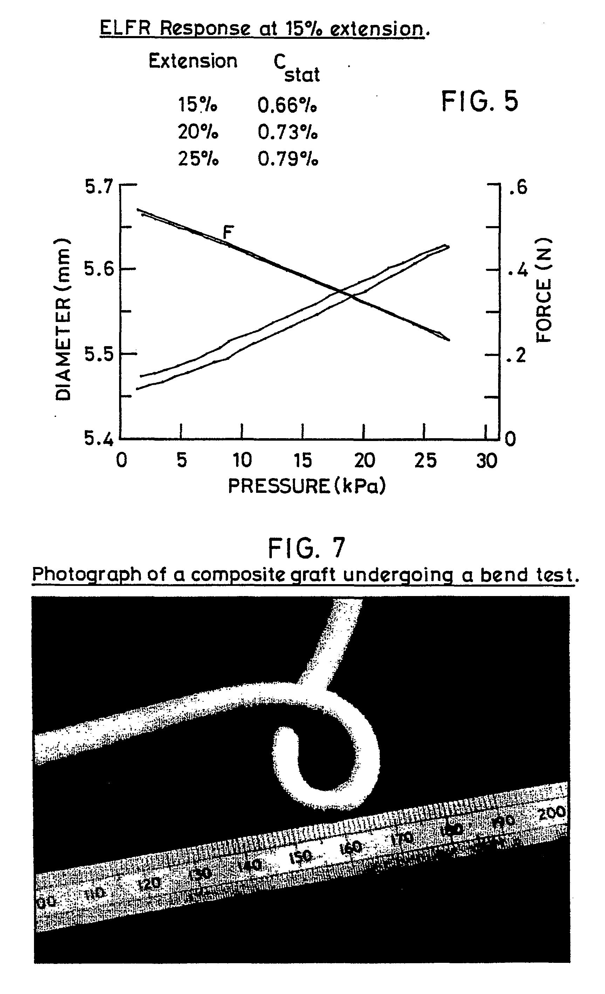

A graph of the change in diameter as a function of internal pressure at I5% axial strain is shown in Figure 5. The graph is essentially linear with only a small hysteresis. The compliance was calculated by measuring the external diameter at I20mm Hg and subtracting it from the diameter at 80mm Hg. This figure was divided by the diameter at I00mm Hg. The static compliance for each of the three experiments was:-
at I5% extension 0.66% compliance
at 20% extension 0.73% compliance
at 25% extension 0.79% compliance

AXIAL EXTENSION UNDER PRESSURE

Owing to the decrease in the axial (longitudinal) Young's modulus of the Fig. 2 mode structure, the specimens will elongate when subjected to internal pressure. This extension was measured for a Fig. 2 mode structure and for a Fig. I mode structure of similar dimensions, the dimensions being

|  | Fig. 2 mode structure | : | Fig. 1 mode structure |
|---|---|---|---|
| Int. Diameter | 4.62mm | | 3.74mm |
| Ext. Diameter | 5.4 mm | | 4.46mm |
| Wall thickness | 0.39mm | | 0.36mm |

At 200mm Hg pressure, a Fig. 2 mode structure increases in length by I5% compared with less than 4% for a wholly microfibrous, standard specimen made in the Fig. I mode. A I5% increase is thought to be within clinically acceptable limits.

## BENDING CHARACTERISTICS OF THE FIG. 2 MODE STRUCTURE.

The bending characteristics of two typical specimens have been determined at zero and 80mm Hg internal pressure. The measurements are compared with results from Fig. I mode structures of similar dimensions. The term "bending diameter" describes the diameter of the smallest circle around which an unsupported structure will bend without kinking.

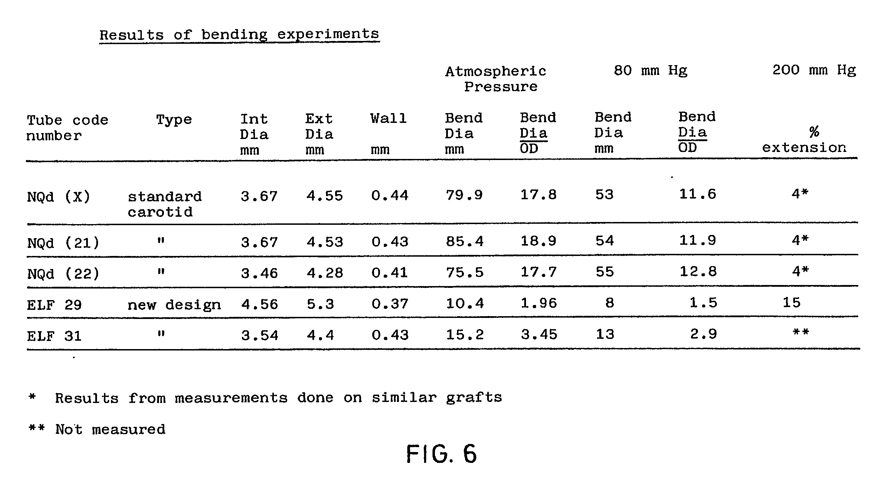

Results of the bending experiments are set out in the table of Figure 6. The mean bending diameter of a Fig. I mode structure is 8cm. A structure of similar gross dimensions but modified by the inclusion of circumferentially aligned macrofibres by the Fig. 2 mode of operation is I.28cm. Figure 7 is a photograph of a similar specimen being tested and illustrates the advantageous bending characteristics.

When the specimens were tested under an imposed internal pressure of 80mm of mercury, the mean bending diameters were 5.4cm and I.0cm respectively.

It will be appreciated that the apparatus described and the types of structure made can be varied significantly depending on the desired characteristics. It will, of course, be understood that the present invention has been described above purely by way of example and modifications of detail can be made within the scope of the invention.

## Claims

1. A tubular fibrous structure comprising small diameter fibres having a diameter in the range 0.5μm to 2μm, and substantially larger diameter fibres having a diameter in the range 2μm to 15μm, said smaller diameter fibres being randomly oriented in the fibrous structure, said larger diameter fibres being embedded in a matrix of said small diameter fibres and said larger diameter fibres being generally oriented circumferentially with respect to the longitudinal axis of said tubular structure.

2. A tubular fibrous structure as claimed in Claim 1 including a multiplicity of elongate voids (16) extending generally circumferentially with respect to the longitudinal axis of the structure.

3. A tubular fibrous structure as claimed in Claim 1 or Claim 2 wherein the ratio of the diameters of the larger diameter fibres to the diameters of the small diameter fibres is less than 30:1.

4. A tubular fibrous structure as claimed in any one of Claims 1 to 3 including at least one layer of fibres all of said small diameter.

5. A tubular fibrous structure as claimed in Claim 4 having a layer of fibres all of said small diameter on the inside of the structure.

6. A tubular fibrous structure as claimed in Claim 5 wherein the layer of fibres of said small diameter has a thickness in the range 10μm to 60μm.

7. A tubular fibrous structure as claimed in Claim 6 wherein the layer of fibres of said small diameter has a thickness of 40μm.

8. A tubular fibrous structure as claimed in any one of Claims 4 to 7 having a layer of fibres of said small diameter on the outside of said structure.

9. A tubular fibrous structure as claimed in Claim 8 wherein the outside layer has a thickness in the range 10μm to 60μm.

10. A tubular fibrous structure as claimed in Claim 9 wherein the outside layer has a thickness of 40μm.

11. A tubular fibrous structure as claimed in any one of Claims 4 to 10 wherein the larger diameter fibres in the matrix of said small diameter fibres lie in a layer of thickness in the range 300μm to 2000μm.

12. A tubular fibrous structure wherein at least an axial portion thereof is a tubular fibrous structure as claimed in any preceding Claim, the complete tubular fibrous structure having been made continuously by electrostatic spinning.

13. A tubular fibrous structure as claimed in Claim 1, wherein said tubular structure has open areas or voids (16) between the larger diameter fibres, said open areas or voids (16) extending in the circumferential direction of the tubular structure whereby said structure has a low modulus in both compression and extension in the axial direction of said tubular structure.

14. A tubular structure according to Claim 12 wherein the inner surface of the structure is predominantly small diameter fibres to provide a smooth inner surface.

15. A tubular structure according to Claim 13 or Claim 14 wherein the outer surface of the structure is predominantly larger diameter fibres and voids (16).

16. A tubular structure according to Claim 13 or Claim 14 wherein the open spaces or voids (16) are disposed on the outer surface of the structure.

17. A tubular structure according to any one of Claims 13 to 16 wherein the larger diameter fibres form circumferential reinforcing hoops, said hoops being disposed in spaced apart relationship along the length of the graft.

18. A tubular structure according to any one of Claims 13 to 17 wherein the inner surface of the structure is relatively smooth and the outer surface of the structure has circumferentially disposed spaced apart ridges.

19. A tubular structure according to any one of Claims 13 to 18 wherein the open spaces or voids (16)

EP 0 223 374 B1

have a tapered depth with the wide portion of said spaces or voids (16) being on the outer surface of the tubular structure.

20. A method of electrostatically spinning a tubular fibrous structure using an electrostatically charged, spinning mandrel (11) and an electrostatically charged grid means (12, 13, 14, 15) in the region of the mandrel (11) to produce an electrostatic field, and means (10) for introducing fibreizable material into said electrostatic field, which method comprises the steps of introducing into said electrostatic field a fibreizable material, collecting on said mandrel (11) a first portion of said liquid in the form of fibres attracted directly to the mandrel (11) and a second portion of said liquid in the form of fibres having been deflected towards said grid means (12, 13, 14, 15) to follow a longer path to said mandrel (11) than the fibres from said first portion of liquid, whereby a tubular fibrous structure as claimed in Claim 1 is produced.

21. A method of electrostatically spinning a tubular fibrous structure as claimed in Claim 20 wherein said fibres from said second portion of said liquid are collected on said mandrel (11) after at least some of said second portion has contacted said grid means (12, 13, 14, 15).

22. A method as claimed in Claim 20 or Claim 21 wherein the fibres from said first and second portions are collected simultaneously.

23. A method as claimed in any one of Claims 20 to 22 which method further comprises the step of selecting a desired potential for the mandrel (11) and selecting a desired potential for the grid means (12, 13, 14, 15), prior to introduction of the fibreizable material into the electrostatic field.

24. A method as claimed in Claim 23 wherein the grid means (12, 13, 14, 15) is selected to have a higher potential than the mandrel (11).

25. A method as claimed in any one of Claims 20 to 24 which method further comprises the steps of arranging the grid means (12, 13, 14, 15) such that the electrostatically charged surface of the grid means (12, 13, 14, 15) nearest the fibreizable material introduction means (10) lies no nearer thereto than the mandrel (11).

26. A method as claimed in any one of Claims 20 to 25 further comprising the steps of starting the electrostatic spinning process with the mandrel (11) at a first mandrel voltage and the grid means (12, 13, 14, 15) at a first grid voltage, and varying the mandrel and grid voltages to cause a variation in the diameter and orientation of at least a portion of the fibres forming the tubular fibrous structure.

27. A method as claimed in Claim 26 wherein the first mandrel voltage and the first grid voltage are such as to produce fibres of a first diameter generally randomly orientated, and the mandrel and grid voltages are varied by increasing the potential of the grid means relative to the mandrel to produce fibres of a larger diameter than the first diameter as well as fibres of the first diameter, the fibres of the larger diameter tending to be orientated generally circumferentially with respect to the longitudinal axis of the mandrel (11).

28. A method as claimed in Claim 26 or Claim 27 comprising a further step of returning the mandrel (11) to the first mandrel voltage and the grid means (12, 13, 14, 15) to the first grid voltage for a period at the end of the process.

29. A method as claimed in Claim 27 or Claim 28 as dependent on Claim 27 comprising the step of arranging and electrostatically charging the grid means (12, 13, 14, 15) such that a leading edge of the grid means (12, 13, 14, 15) lies forward of the mandrel (11) with respect to the fibreizable material introduction means (10) where fibres of said first diameter are required, and such that a leading edge of the grid means (13, 15) lies no nearer the fibreizable material introduction means (10) than the mandrel when fibres of said second diameter are required.

30. A method as claimed in Claim 29 where the grid means comprise a first pair of coplanar grids (12, 13) or plates on one side of the mandrel (11) and a second pair of coplanar grids (14, 15) or plates on the other side of the mandrel (11) and in a plane parallel to the first pair of coplanar grids or plates, one grid or plate (12, 14) of each coplanar pair lying forward of the mandrel (11) with respect to the fibreizable material introduction means (10), and the other grid or plate (13, 15) of each coplanar pair lying no nearer the fibreizable material introduction means (10) than the mandrel (11), which method comprises the steps of electrostatically charging both grids or plates of each pair when fibres of said first diameter are required and electrostatically charging the second grid or plate (13, 15) only of each pair when fibres of a diameter larger than said first diameter are required.

31. A method as claimed in any one of Claims 20 to 30 wherein the mandrel (11) is charged to a potential in the range 6kV to 20kV.

32. A method as claimed in any one of Claims 20 to 31 wherein, for a mandrel of 4mm diameter, the mandrel (11) is charged to 7kV and the grid means (13, 15) to 9.2kV to produce fibres of different diameters.

33. A method as claimed in any one of Claims 20 to 32 comprising the step of traversing the fibre introduction means (10) along the length of the mandrel (11) and varying the electrostatic potentials of the mandrel (11) and the grid means (12, 13, 14, 15) as the fibreizable material introduction means (10) moves relative to the mandrel (11) to produce different electrostatic fields for fibre collection and hence different fibrous structures at different axial locations on the mandrel (11).

34. Apparatus for electrostatically spinning a tubular fibrous structure, which apparatus comprises a mandrel (11) to act as a collector for electrostatically spun fibres, means for electrostatically charging the mandrel and for varying the electrostatic charge thereon, means for rotating the mandrel, grid means (13, 15) in the region of the mandrel (11), means for electrostatically charging the grid means (13, 15)

7

and for varying the electrostatic charge thereon, and means (10) for introducing a fibreizable material into the electrostatic field, the surface of the grid means (13, 15) nearest to the fibreizable material introduction means lying no nearer thereto than the mandrel (11).

35. Apparatus as claimed in Claim 34 wherein the grid means comprises a pair of parallel plates or grids (13, 15) arranged one each side of the mandrel (11).

36. Apparatus as claimed in Claim 35 wherein leading edges of the plates (13, 15) nearest the fibreizable material introduction means (10) are parallel.

37. Apparatus as claimed in Claim 36 wherein the parallel leading edges lie in a plane passing through or at least adjacent the mandrel (11).

38. Apparatus as claimed in any one of Claims 34 to 37 further comprising secondary grid means (12, 14) extending forwardly of the grid means (13, 15) with respect to the fibreizable material introduction means.

39. Apparatus as claimed in Claim 38 wherein the secondary grid means (12, 14) comprise a pair of parallel plates (12, 14).

40. Apparatus as claimed in Claim 38 or Claim 39 comprising means for electrostatically charging the grid means (13, 15) and the secondary grid means (12, 14) together, or the grid means (13, 15) alone.

41. Apparatus as claimed in any one of Claims 34 to 40 comprising microprocessor means for controlling variation of the voltages in accordance with a desired sequence to produce a tubular fibrous structure having desired characteristics.

## Patentansprüche

1. Röhrenförmige Faserstruktur mit Fasern mit einem kleinen Durchmesser im Bereich von 0,5 μm bis 2 μm und Fasern mit einem wesentlich größeren Durchmesser im Bereich von 2 μm bis 15 μm, wobei die Fasern mit dem kleineren Durchmesser statistisch in der Faserstruktur orientiert sind, die Fasern mit dem größeren Durchmesser in einer Matrix der Fasern mit dem kleinen Durchmesser eingebettet sind und im allgemeinen ringförmig in bezug auf die Längsachse der röhrenförmigen Struktur orientiert sind.

2. Röhrenförmige Faserstruktur nach Anspruch 1 mit einer Vielzahl von länglichen Hohlräumen (16), die sich im allgemeinen ringförmig in bezug auf die Längsachse der Struktur erstrecken.

3. Röhrenförmige Faserstruktur nach Anspruch 1 oder 2, in der das Verhältnis der Durchmesser der Fasern mit dem größeren Durchmesser zu denen der Fasern mit dem kleinen Durchmesser unter 30:1 beträgt.

4. Röhrenförmige Faserstruktur nach einem der Ansprüche 1 bis 3 mit mindestens einer Schicht von Fasern, die alle den kleinen Durchmesser haben.

5. Röhrenförmige Faserstruktur nach Anspruch 4 mit einer Schicht von Fasern, die alle den kleinen Durchmesser haben auf der Innenseite der Struktur.

6. Röhrenförmige Faserstruktur nach Anspruch 5, in der die Schicht der Fasern mit dem kleinen Durchmesser eine Dicke im Bereich von 10 μm bis 60 μm hat.

7. Röhrenförmige Faserstruktur nach Anspruch 6, in der die Schicht der Fasern mit dem kleinen Durchmesser eine Dicke von 40 μm hat.

8. Röhrenförmige Faserstruktur nach einem der Ansprüche 4 bis 7 mit einer Schicht von Fasern mit dem kleinen Durchmesser auf der Außenseite der Struktur.

9. Röhrenförmige Faserstruktur nach Anspruch 8, in der die Außenschicht eine Dicke im Bereich von 10 μm bis 60 μm hat.

10. Röhrenförmige Faserstruktur nach Anspruch 9, in der die Außenschicht eine Dicke von 40 μm hat.

11. Röhrenförmige Faserstruktur nach einem der Ansprüche 4 bis 10, in der die Fasern mit dem größeren Durchmesser in der Matrix der Fasern mit dem kleinen Durchmesser in einer Schicht mit einer Dicke im Bereich von 300 μm bis 2000 μm liegen.

12. Röhrenförmige Faserstruktur, in der zumindest ein Axialbereich eine röhrenförmige Faserstruktur wie in einem der vorstehenden Ansprüche ist und die vollständige röhrenförmige Faserstruktur kontinuierlich durch elektrostatisches Spinnen hergestellt worden ist.

13. Röhrenförmige Faserstruktur nach Anspruch 1, in der die röhrenförmige Struktur offene Bereiche oder Hohlräume (16) zwischen den Fasern mit dem größeren Durchmesser hat, die offenen Bereiche oder Hohlräume (16) sich in Umfangsrichtung der röhrenförmigen Struktur erstrecken, wodurch die Struktur einen niedrigen sowohl Druck- wie auch Dehnmodul in der Axialrichtung der röhrenförmigen Struktur hat.

14. Röhrenförmige Faserstruktur nach Anspruch 12, in der die Innenfläche der Struktur im wesentlichen aus Fasern mit kleinem Durchmesser besteht und glatt ist.

15. Röhrenförmige Faserstruktur nach Anspruch 13 oder 14, in der die Außenfläche der Struktur im wesentlichen aus Fasern mit größerem Durchmesser und Hohlräumen (16) besteht.

16. Röhrenförmige Faserstruktur nach Anspruch 13 oder 14, in der die offenen Bereiche oder Hohlräume (16) an der Außenfläche der Struktur angeordnet sind.

17. Röhrenförmige Faserstruktur nach einem der Ansprüche 13 bis 16, in dem die Fasern mit größerem Durchmesser verstärkende Umfangsringe bilden, die in miteinander in Verbindung stehenden Zwischenräumen entlang der Länge des Pfropfprodukts angeordnet sind.

18. Röhrenförmige Faserstruktur nach einem der Ansprüche 13 bis 17, in der die Innenfläche der Struktur relativ glatt ist und die Außenfläche der Struktur ringförmig und im Abstand voneinander angeordnete Rillen aufweist.

19. Röhrenförmige Faserstruktur nach einem der Ansprüche 13 bis 18, in der die offenen Bereiche oder Hohlräume (16) eine kegelförmige Tiefe haben, wobei der weite Bereich der offenen Bereiche oder Hohlräume (16) an der Außenfläche der röhrenförmigen Struktur liegt.

20. Verfahren zum elektrostatischen Spinnen einer röhrenförmigen Faserstruktur unter Verwendung eines elektrostatisch geladenen Spinndorns (11) und einer elektrostatisch geladenen Gittervorrichtung (12, 13, 14, 15) im Bereich des Dorns (11) zur Herstellung eines elektrostatischen Feldes und einer Vorrichtung (10) zum Einführen eines zu Fasern verarbeitbaren Materials in das elektrostatische Feld, mit folgenden Stufen:
Einführen eines zu Fasern verarbeitbaren Materials in das elektrostatische Feld,
Sammeln an dem Dorn (11) eines ersten Teils der Flüssigkeit in Form von zum Dorn (11) direkt angezogenen Fasern und eines zweiten Teils der Flüssigkeit in Form von Fasern, die zu der Gittervorrichtung (12, 13, 14, 15) abgelenkt worden sind und somit einen längeren Weg zum Dorn (11) zurücklegen als die Fasern des ersten Flüssigkeitsteils, wodurch eine röhrenförmige Faserstruktur nach Anspruch 1 erhalten wird.

21. Verfahren zum elektrostatischen Spinnen einer röhrenförmigen Faserstruktur nach Anspruch 20, in dem die Fasern aus dem zweiten Flüssigkeitsteil an dem Dorn (11) gesammelt werden, nachdem mindestens ein Teil des zweiten Teils die Gittervorrichtung (12, 13, 14, 15) kontaktiert hat.

22. Verfahren nach Anspruch 20 oder 21, in dem die Fasern aus dem ersten und dem zweiten Teil gleichzeitig gesammelt werden.

23. Verfahren nach einem der Ansprüche 20 bis 22 mit einer weiteren Stufe, in der ein gewünschtes Potential für den Dorn (11) und ein gewünschtes Potential für die Gittervorrichtung (12, 13, 14, 15) gewählt wird, bevor das zu Fasern verarbeitbare Material in das elektrostatische Feld eingeführt wird.

24. Verfahren nach Anspruch 23, in dem die Gittervorrichtung (12, 13, 14, 15) ein höheres Potential als der Dorn (11) hat.

25. Verfahren nach einem der Ansprüche 20 bis 24 mit einer weiteren Stufe, in der die Gittervorrichtung (12, 13, 14, 15) so angeordnet wird, daß die elektrostatisch geladene Fläche der Gittervorrichtung (12, 13, 14, 15), die der Einführvorrichtung (10) des zu Fasern verarbeitbaren Materials am nächsten liegt, ihr nicht näher ist als der Dorn (11).

26. Verfahren nach einem der Ansprüche 20 bis 25 mit folgenden weiteren Stufen:
Beginnen des elektrostatischen Spinnverfahrens mit dem Dorn (11) bei einer ersten Dornspannung und der Gittervorrichtung (12, 13, 14, 15) bei einer ersten Gitterspannung, und
Ändern der Dorn- und Gitterspannungen, um so eine Änderung der Durchmesser und der Orientierung mindestens eines Teils der die röhrenförmige Faserstruktur bildenden Fasern zu verursachen.

27. Verfahren nach Anspruch 26, in dem die erste Dornspannung und die erste Gitterspannung so sind, daß Fasern mit einem ersten Durchmesser, die im allgemeinen statistisch orientiert sind, erhalten werden, und die Dorn- und Gitterspannungen verändert werden durch Erhöhen des Potentials der Gittervorrichtung in bezug auf den Dorn, wodurch Fasern mit einem größeren Durchmesser als der erste Durchmesser sowie Fasern des ersten Durchmessers erhalten werden, die Fasern mit dem größeren Durchmesser eher im allgemeinen ringförmig in bezug auf die Längsachse des Dorns (11) orientiert sind.

28. Verfahren nach Anspruch 26 oder 27 mit einer weiteren Stufe, in der der Dorn (11) zur ersten Dornspannung und die Gittervorrichtung (12, 13, 14, 15) zur ersten Gitterspannung während einer Zeit am Ende des Verfahrens zurückgebracht werden.

29. Verfahren nach Anspruch 27 oder 28, bezogen auf Anspruch 27, mit einer Stufe, in der die Gittervorrichtung (12, 13, 14, 15) so angeordnet und elektrostatisch geladen wird, daß eine Vorderkante der Gittervorrichtung (12, 13, 14, 15) vor dem Dorn (11) in bezug auf die Einführvorrichtung (10) des zu Fasern verarbeitbaren Materials liegt, wenn Fasern des ersten Durchmessers benötigt werden, und so, daß eine Vorderkante der Gittervorrichtung (13, 15) der Einführvorrichtung (10) des zu Fasern verarbeitbaren Materials nicht näher liegt als der Dorn, wenn Fasern des zweiten Durchmessers benötigt werden.

30. Verfahren nach Anspruch 29, in dem die Gittervorrichtung ein erstes Paar von koplanaren Gittern (12, 13) oder Platten auf einer Seite des Dorns (11) und ein zweites Paar von koplanaren Gittern (14, 15) oder Platten auf der anderen Seite des Dorns (11) und in einer zum ersten Paar der koplanaren Gitter oder Platten parallelen Ebene umfaßt, wobei ein Gitter oder eine Platte (12, 14) jedes koplanaren Paars vor dem Dorn (11) in bezug auf die Einführvorrichtung (10) des zu Fasern verarbeitbaren Materials liegt und das andere Gitter oder die andere Platte (13, 15) jedes koplanaren Paars der Einführvorrichtung (10) des zu Fasern verarbeitbaren Materials nicht näher liegt als der Dorn (11), beide Gitter oder Platten jedes Paares elektrostatisch geladen werden, wenn Fasern des ersten Durchmessers benötigt werden, und das zweite Gitter oder die zweite Platte (13, 15) jedes Paares nur dann elektrostatisch geladen wird, wenn Fasern mit einem größeren Durchmesser als der erste Durchmesser benötigt werden.

31. Verfahren nach einem der Ansprüche 20 bis 30, in dem der Dorn (11) auf ein Potential im Bereich von 6 kV bis 20 kV geladen wird.

32. Verfahren nach einem der Ansprüche 20 bis 31, in dem für einen Dorn mit einem Durchmesser von 4 mm der Dorn (11) auf 7 kV und die Gittervorrichtung (13, 15) auf 9,2 kV geladen werden, um Fasern mit verschiedenen Durchmessern herzustellen.

33. Verfahren nach einem der Ansprüche 20 bis 32 mit einer Stufe, in der die Fasereinführvorrichtung (10) entlang der Länge des Dorns (11) quer bewegt wird und die elektrostatischen Potentiale des Dorns (11) und der Gittervorrichtung (12, 13, 14, 15) mit der Bewegung der Einführvorrichtung (10) des zu Fasern verarbeitbaren Materials in bezug auf den Dorn (11) verändert werden, um verschiedene elektrostatische Felder für die Faseransammlung und folglich verschiedene Faserstrukturen an verschiedenen Axialstellen des Dorns (11) zu erzeugen.

34. Vorrichtung für das elektrostatische Spinnen einer röhrenförmigen Faserstruktur mit einem Dorn (11) zum Sammeln von elektrostatisch gesponnenen Fasern, einer Vorrichtung zum elektrostatischen Laden des Dorns und zum Verändern der darauf angebrachten elektrostatischen Ladung, einer Vorrichtung zum Drehen des Dorns, der Gittervorrichtung (13, 15) im Bereich des Dorns (11), einer Vorrichtung zum elektrostatischen Laden der Gittervorrichtung (13, 15) und zum Ändern der darauf aufgebrachten elektrostatischen Ladung und einer Vorrichtung (10) zum Einführen eines zu Fasern verarbeitbaren Materials in das elektrostatische Feld, wobei die Fläche der Gittervorrichtung (13, 15) die der Einführvorrichtung für das zu Fasern verarbeitbare Material am nächsten liegt, ihr nicht näher als der Dorn (11) ist.

35. Vorrichtung nach Anspruch 34, in dem die Gittervorrichtung ein Paar paralleler Platten oder Gitter (13, 15), die auf jeder Seite des Dorns (11) angeordnet sind, umfaßt.

36. Vorrichtung nach Anspruch 35, in der Vorderkanten der Platten (13, 15), die der Einführvorrichtung (10) des zu Fasern verarbeitbaren Materials am nächsten liegen, parallel sind.

37. Vorrichtung nach Anspruch 36, in der die parallelen Vorderkanten in einer Ebene liegen, die durch den Dorn (11) geht oder ihm zumindest nahe ist.

38. Vorrichtung nach einem der Ansprüche 34 bis 37 mit einer Nebengittervorrichtung (12, 14), die vor der Gittervorrichtung (13, 15) in bezug auf die Einführvorrichtung für das zu Fasern verarbeitbare Material liegt.

39. Vorrichtung nach Anspruch 38, in der die Nebengittervorrichtung (12, 14) ein Paar paralleler Platten (12, 14) umfaßt.

40. Vorrichtung nach Anspruch 38 oder 39 mit einer Vorrichtung zum elektrostatischen Laden der Gittervorrichtung (13, 15) und der Nebengittervorrichtung (12, 14) zusammen oder der Gittervorrichtung (13, 15) alleine.

41. Vorrichtung nach einem der Ansprüche 34 bis 40 mit einer Mikroprozessor-Vorrichtung zum Regeln der Spannungsänderungen entsprechend einer gewünschten Folge zur Herstellung einer röhrenförmigen Faserstruktur mit den gewünschten Eigenschaften.

**Revendications**

1. Une structure fibreuse tubulaire comprenant des fibres de petit diamètre présentant un diamètre compris dans la plage de 0,5 µm à 2 µm, et des fibres de diamètre sensiblement plus important présentant un diamètre compris dans la plage de 2 µm à 15 µm, lesdites fibres de plus petit diamètre étant orientées de façon aléatoire dans la structure fibreuse, lesdites fibres de plus grand diamètre étant noyées dans une matrice desdites fibres de petit diamètre et lesdites fibres de plus grand diamètre étant orientées de façon généralement circonférentielle par rapport à l'axe longitudinal de ladite structure tubulaire.

2. Une structure tubulaire fibreuse selon la revendication 1, comprenant une pluralité de vides allongés (16) s'étendant de façon généralement circonférentielle par rapport à l'axe longitudinal de la structure.

3. Une structure tubulaire fibreuse selon la revendication 1 ou la revendication 2, dans laquelle le rapport des diamètres des fibres de plus grand diamètre aux diamètres des fibres de petit diamètre est inférieur à 30:1.

4. Une structure tubulaire fibreuse selon l'une quelconque des revendications 1 à 3, comprenant au moins une couche de fibres qui sont toutes de petit diamètre.

5. Une structure tubulaire fibreuse selon la revendication 4, comportant sur l'intérieur de la structure une couche de fibres qui sont toutes dudit petit diamètre.

6. Une structure tubulaire fibreuse selon la revendication 5, dans laquelle la couche de fibres dudit petit diamètre est d'une épaisseur comprise dans la plage de 10 µm à 60 µm.

7. Une structure tubulaire fibreuse selon la revendication 6, dans laquelle la couche de fibres dudit petit diamètre est d'une épaisseur de 40 µm.

8. Une structure tubulaire fibreuse selon l'une quelconque des revendications 4 à 7, comportant une couche de fibres dudit petit diamètre sur l'extérieur de ladite structure.

9. Une structure tubulaire fibreuse selon la revendication 8, dans laquelle la couche extérieure est d'une épaisseur comprise dans la plage de 10 µm à 60 µm.

10. Une structure tubulaire fibreuse selon la revendication 9, dans laquelle la couche extérieure est d'une épaisseur de 40 µm.

11. Une structure tubulaire fibreuse selon l'une quelconque des revendications 4 à 10 dans laquelle les fibres de plus grand diamètre situées dans la matrice de fibres dudit petit diamètre sont situées dans une couche d'épaisseur comprise dans la plage de 300 µm à 2000 µm.

12. Une structure tubulaire fibreuse dans laquelle au moins une partie axiale est une structure tubulaire fibreuse selon l'une quelconque des précédentes revendications, la structure tubulaire fibreuse complète ayant été réalisée de façon continue par centrifugation électrostatique.

13. Une structure tubulaire fibreuse selon la revendication 1, dans laquelle ladite structure tubulaire fibreuse comporte des zones ouvertes ou vides (16) entre les fibres de plus grand diamètre, lesdites zones ouvertes ou vides (16) s'étendant dans la direction circonférentielle de la structure tubulaire, grâce à quoi ladite structure possède un module faible à la fois en compression et en allongement dans la direction axiale de ladite structure tubulaire.

14. Une structure tubulaire selon la revendication 12, dans laquelle la surface intérieure de la structure est de façon prédominante constituée de fibres de petit diamètre pour réaliser une surface intérieur lisse.

15. Une structure tubulaire selon la revendication 13 ou la revendication 14, dans laquelle la surface extérieure de la structure est de façon prédominante constituée de fibres de plus grand diamètre et de vides (16).

16. Une structure tubulaire selon la revendication 13 ou la revendication 14, dans laquelle les espaces ouverts ou vides (16) sont disposés sur la surface extérieure de la structure.

17. Une structure tubulaire selon l'une quelconque des revendications 13 à 16, dans laquelle les fibres de plus grand diamètre forment des cerceaux de renforcement circonférentiels, lesdits cerceaux étant disposés dans une relation espacée l'un de l'autre le long de la longueur de la greffe.

18. Une structure tubulaire selon l'une quelconque des revendications 13 à 17, dans laquelle la surface intérieure de la structure est relativement lisse et la surface extérieure de la structure possède des sillons espacés entre eux, disposés circonférentiellement.

19. Une structure tubulaire selon l'une quelconque des revendications 13 à 18, dans laquelle les espaces ouverts ou vides (16) se rétrécissent en profondeur, la partie large desdits espaces ou vides (16) étant sur la surface extérieure de la structure tubulaire.

20. Un procédé de centrifugation électrostatique d'une structure fibreuse tubulaire utilisant un mandrin de centrifugation (11) chargé électrostatiquement et des moyens à grille (12, 13, 14, 15) chargés électrostatiquement dans la zone du mandrin (11) pour produire un champ électrostatique, et des moyens (10) pour introduire une matière transformable en fibres dans ledit champ électrostatique, ledit procédé comprenant les étapes consistant à introduire dans ledit champ électrostatique une matière transformable en fibres, recueillir sur ledit mandrin (11) une première partie dudit liquide sous forme de fibres attirées directement vers le mandrin (11) et une deuxième partie dudit liquide sous forme de fibres qui ont été déviées vers lesdits moyens à grille (12, 13, 14, 15) pour suivre, vers ledit mandrin (11), un trajet plus long que les fibres provenant de ladite première partie de liquide, grâce à quoi est produite une structure tubulaire fibreuse selon la revendication 1.

21. Un procédé de centrifugation électrostatique d'une structure tubulaire fibreuse selon la revendication 20, dans lequel lesdites fibres provenant de ladite deuxième partie dudit liquide sont recueillies sur ledit mandrin (11) après qu'au moins une fraction de ladite deuxième partie est venue en contact avec lesdits moyens à grille (12, 13, 14, 15).

22. Un procédé selon la revendication 20 ou la revendication 21, dans lequel les fibres provenant desdites première et deuxième parties sont recueillies simultanément.

23. Un procédé selon l'une quelconque des revendications 20 à 22, procédé qui comprend de plus l'étape consistant à choisir un potentiel souhaité pour le mandrin (11) et à choisir un potentiel souhaité pour les moyens à grille (12, 13, 14, 15), avant d'introduire la matière transformable en fibres dans le champ électrostatique.

24. Un procédé selon la revendication 23, dans lequel les moyens à grille (12, 13, 14, 15) sont choisis de manière à présenter un potentiel plus élevé que le mandrin (11).

25. Un procédé selon l'une quelconque des revendications 20 à 24, le procédé comprenant de plus l'étape consistant à disposer les moyens à grille (12, 13, 14, 15) de telle façon que la surface chargée électrostatiquement des moyens à grille (12, 13, 14, 15) la plus voisine des moyens d'introduction (10) de matière transformable en fibres ne soit pas située plus près de ces moyens que le mandrin (11).

26. Un procédé selon l'une quelconque des revendications 20 à 25, comprenant de plus l'étape consistant à commencer le procédé de centrifugation électrostatique quand le mandrin (11) est à une première tension de mandrin et que les moyens à grille (12, 13, 14, 15) sont à une première tension de grille, et à faire varier les tensions de mandrin et de grille pour provoquer une variation du diamètre et de l'orientation d'au moins une partie des fibres constituant la structure tubulaire fibreuse.

27. Un procédé selon la revendication 26, dans lequel la première tension de mandrin et la première tension de grille sont aptes à produire des fibres d'un premier diamètre orienté généralement de façon aléatoire, et les tensions de mandrin et de grille sont soumises à une variation en augmentant le potentiel des moyens à grille par rapport au mandrin pour produire des fibres d'un diamètre plus grand que le premier diamètre ainsi que des fibres du premier diamètre, les fibres du plus grand diamètre tendant à être orientées généralement de façon circonférentielle par rapport à l'axe longitudinal du mandrin (11).

28. Un procédé selon la revendication 26 ou la revendication 27, comprenant une étape additionnelle consistant à ramener le mandrin (11) à la première tension de mandrin et les moyens à grille (12, 13, 14, 15) à la première tension de grille pendant une période à la fin du processus.

29. Un procédé selon la revendication 27 ou la revendication 28 quand elle dépend de la revendication 27, comprenant l'étape consistant à disposer et à charger électrostatiquement les moyens à grille (12, 13, 14, 15) de telle façon qu'un bord avant des moyens à grille (12, 13, 14, 15) soit situé en avant du mandrin (11) par rapport aux moyens d'introduction de la matière transformable en fibres (10), dans le cas où les fibres dudit premier diamètre sont demandées, et tel qu'un bord avant des moyens à grille (13, 15) ne soit pas situé plus près des moyens d'introduction de la matière transformable en fibres (10) que le mandrin dans le cas où des fibres dudit deuxième diamètre sont demandées.

30. Un procédé selon la revendication 29, dans lequel les moyens à grille comprennent une première paire de grilles ou plaques coplanaires (12, 13) sur un côté du mandrin (11) et une deuxième paire de grilles ou plaques coplanaires (14, 15) sur l'autre côté du mandrin (11) et dans un plan parallèle à la première paire de grilles ou de plaques coplanaires, une grille ou plaque (12, 14) de chaque paire coplanaire étant située en avant du mandrin (11) par rapport aux moyens d'introduction de la matière transformable en fibres (10), et l'autre grille ou plaque (13, 15) de chaque paire coplanaire n'étant pas située plus près des moyens d'introduction de matière transformable en fibres (10) que le mandrin (11), ce procédé comprenant les étapes consistant à charger électrostatiquement les deux grilles ou plaques de chaque paire lorsque des fibres dudit premier diamètre sont demandées et à ne charger électrostatiquement que la deuxième grille ou plaque (13, 15) de chaque paire lorsque des fibres d'un diamètre plus grand que ledit premier diamètre sont demandées.

31. Un procédé selon l'une quelconque des revendications 20 à 30, dans lequel le mandrin (11) est chargé à un potentiel situé dans la plage de 6 kV à 20 kV.

32. Un procédé selon l'une quelconque des revendications 20 à 31, dans lequel, pour un mandrin d'un diamètre de 4 mm, le mandrin (11) est chargé à 7 kV et les moyens à grille (13, 15) à 9,2 kV pour produire des fibres de diamètres différents.

33. Un procédé selon l'une quelconque des revendications 20 à 32, comprenant l'étape consistant à amener les moyens d'introduction (10) de fibres le long de la longueur du mandrin (11) et à faire varier les potentiels électrostatiques du mandrin (11) et les moyens à grille (12, 13, 14, 15) lorsque les moyens d'introduction (10) de matière transformable en fibres se déplacent par rapport au mandrin (11) pour produire des champs électrostatiques différents pour recueillir les fibres, et par conséquent des structures fibreuses différentes à des emplacements axiaux différents du mandrin (11).

34. Appareil pour centrifuger électrostatiquement une structure fibreuse tubulaire, l'appareil comprenant un mandrin (11) destiné à agir pour recueillir des fibres centrifugées électrostatiquement, des moyens pour charger électrostatiquement le mandrin et pour faire varier la charge électrostatique qui sur celui-ci, des moyens pour faire tourner mandrin, des moyens à grille (13, 15) dans la zone du mandrin (11), des moyens pour charger électrostatiquement les moyens à grille (13, 15) et pour faire varier la charge électrostatique sur ceux-ci, et des moyens (10) pour introduire une matière transformable en fibres dans le champ électrostatique, la surface des moyens à grille (13, 15) la plus proche des moyens d'introduction de la matière transformable en fibres n'étant pas plus proche de ceux-ci que le mandrin (11).

35. Appareil selon la revendication 34, dans lequel les moyens à grille comprennent une paire de plaques ou grilles parallèles (13, 15) disposées sur chaque côté du mandrin (11).

36. Appareil selon la revendication 35 dans lequel des bords avant des plaques (13, 15) les plus proches des moyens d'introduction (10) de matière transformable en fibres sont parallèles.

37. Appareil selon la revendication 36, dans lequel les bords avant parallèles sont situés dans un plan passant par le mandrin (11) ou au moins à proximité de celui-ci.

38. Appareil selon l'une quelconque des revendications 34 à 37, comprenant de plus des moyens à grille secondaires (12, 14) s'étendant en avant des moyens de grille (13, 15) par rapport aux moyens d'introduction de matière transformable en fibres.

39. Appareil selon la revendication 38, dans lequel les moyens à grille secondaires (12, 14) comprennent une paire de plaques parallèles (12, 14).

40. Appareil selon la revendication 38 ou la revendication 39, comprenant des moyens pour charger électrostatiquement les moyens à grille (13, 15) et les moyens à grille secondaires (12, 14) en même temps, ou les moyens à grille (13, 15) seuls.

41. Appareil selon l'une quelconque des revendications 34 à 40, comprenant des moyens à microprocesseur pour commander la variation des tensions selon une séquence désirée pour produire une structure fibreuse tubulaire possédant des caractéristiques souhaitées.

FIG. 1

FIG. 2

SEM of the microstructure of a composite graft.
Scale: 1mm. = 1µm.

FIG. 3

FIG. 4

EP 0 223 374 B1

## ELFR Response at 15% extension.

| Extension | $C_{stat}$ |
|-----------|------------|
| 15% | 0.66% |
| 20% | 0.73% |
| 25% | 0.79% |

## FIG. 5

## FIG. 7

## Photograph of a composite graft undergoing a bend test.

Results of bending experiments

| | | | | | Atmospheric Pressure | | 80 mm Hg | | 200 mm Hg |
|---|---|---|---|---|---|---|---|---|---|
| Tube code number | Type | Int Dia mm | Ext Dia mm | Wall mm | Bend Dia mm | Bend $\frac{Dia}{OD}$ | Bend Dia mm | Bend $\frac{Dia}{OD}$ | % extension |
| NQd (X) | standard carotid | 3.67 | 4.55 | 0.44 | 79.9 | 17.8 | 53 | 11.6 | 4* |
| NQd (21) | " | 3.67 | 4.53 | 0.43 | 85.4 | 18.9 | 54 | 11.9 | 4* |
| NQd (22) | " | 3.46 | 4.28 | 0.41 | 75.5 | 17.7 | 55 | 12.8 | 4* |
| ELF 29 | new design | 4.56 | 5.3 | 0.37 | 10.4 | 1.96 | 8 | 1.5 | 15 |
| ELF 31 | " | 3.54 | 4.4 | 0.43 | 15.2 | 3.45 | 13 | 2.9 | ** |

\* Results from measurements done on similar grafts

\** Not measured

## FIG. 6

EP 0 223 374 B1